# EUROPEAN PATENT SPECIFICATION

(11) **EP 0 919 188 B1**
(45) Date of publication and mention of the grant of the patent: **16.06.2004**
(21) Application number: 98309612.4
(22) Date of filing: 24.11.1998
(51) Int. Cl.: A61B 8/00

(54) **Ultrasonic probe connector enclosure**
Gehäuse für Steckverbinder für eine Ultraschallsonde
Boîtier pour connecteur d'une sonde à ultrasons

(30) Priority: 25.11.1997 US 66946 P
(43) Date of publication of application: 02.06.1999
(60) Divisional application: 03078425.0
(73) Proprietor: ATL Ultrasound, Inc., Bothell, Washington 98021 (US)
(72) Inventor: Green, Dustin J., Carnation, WA 98014 (US); Nordgren, Tim, Bbothell, WA 98021 (US); Heigel, Wayne, Issaquah, WA 98029 (US)
(74) Representative: Lottin, Claudine

(56) References cited:
- EP-A- 0 687 034
- EP-A- 0 709 924
- DE-A- 19 511 394
- JP-A- 38 086
- US-A- 5 678 551

## Description

This invention relates to improvements in connector enclosures and housings for ultrasonic diagnostic imaging system probes, in particular, to connector enclosures and housings used for protecting sensitive electronic components during the sterilization process of such probes.

Ultrasonic diagnostic imaging systems are in widespread use for performing ultrasonic imaging and measurements of the human body through the use of probes which may be placed internal or external to the body being measured. Such probes are used to view the internal structure of a body with an array of transducer elements that transmits pulses or beams of energy into the body and receive returning pulses of energy as they are reflected from internal structures of the body. An ultrasonic probe assembly is typically comprised of a scan head, which houses the array of transducer elements, a multi-conductor cable, and a multi-pin connector. The pulses or beams of energy that are transmitted from and received by the array of transducer elements are electrically coupled to the ultrasonic diagnostic imaging systems by the cable and connector of the probe assembly.

Intra-operative probes are used in applications such as vascular or implant surgeries where the scanhead is placed inside the body during the surgery. To use the same probe on a different patient after a surgical procedure, the probe must be sterilized. New sterilization techniques require submersion of the probe in a liquid environment. A typical sterilization process requires the placement of the entire probe, including the connector and the cable, into a liquid tight chamber of a sterilization device. During the sterilization process, the chamber is filled with an acidic solution at an elevated temperature. The probe is then subjected to a wash cycle followed by several rinse cycles.

Since intra-operative probe connectors are comprised of moisture sensitive components, such components are subject to damage or corrosion if exposed to the liquid environment of the sterilization process. For example, the electrical characteristics of the pin connectors are important because the signals sent to and from the transducer array are carried through the connector pins. Since the transducer is comprised of multiple numbers of small piezoelectric elements that are generally made of a crystalline material, it is imperative that all of the electrical connections made to the transducer through the connector and cable are reliable. It is essential that no spurious or harmful electrical conditions be introduced or engendered, which may exist if the connector pins or other moisture sensitive components in the connector are exposed to harsh liquids. Accordingly, the sterilization techniques for intra-operative probes require the connectors to be sealed in a liquid tight manner.

To date, techniques for sealing the connectors for sterilization include the use of plugs or caps that are inserted into or over the probe connectors. Such techniques are not reliable because they tend to leak, thus exposing the sensitive electrical components of the connector to a liquid environment. Other techniques for sealing the connector include the use of a cap having a face seal that is placed over the connector pins. That technique, however, is limiting because it only operates with connectors having a specific type of connector shaft.

For example, when such a cap is connected to a connector using a connector shaft, the user must position a member of the shaft in a keyhole in the cap. As the member is rotated inside of the keyhole, the cap and face seal will be drawn into the connector. The use of a connector shaft as an integral part of sealing a connector is described in U.S. Pat. No. 5630,419. Such devices are not reliable, however, because the connector shaft provides only a quarter of turn in rotation to draw the cap down to a sealable position. Thus, if the gasket member becomes less resilient over time or the components used to draw down the cap become worn over time, the chance of leakage increases because a greater draw cannot be provided by the connector shaft.

Other connectors have been designed to accept a cover that seals the connector for submersion in a sterilization liquid. Such connector covers, however, rely on the internal connector assembly for latching the cover to the connector housing. Such a connector is described in U.S. Pat. No. 5678,551. One embodiment of the present invention provides a connector housing designed to accept a cover that seals the connector for submersion in a sterilization liquid without reliance on the internal connector assembly. Moreover, the devices described above may be expensive and require extensive assembly due to the multiplicity of parts. Accordingly, it is desirable to have a simple and reliable device for providing a watertight seal for an ultrasonic probe connector, such that it may be completely submersed in a sterilization liquid without causing damage to the components of the connector.
A waterproof case for a connector is described in the patent EP0687034 A2. This document relates to a waterproof case in which a hood contains a connector to prevent the connector from being exposed to water, with a cap for closing an opening portion of the hood through a mounting linkage. A lever is rotatably mounted to the hood at an end thereof. A locking linkage is rotatably mounted at an end thereof to an intermediate portion, which is between both ends of the lever. When the lever is rotated by operating the other end with the other end of the cap in the state of closing the opening portion, the cap is kept in condition in which the opening portion is closed.
A sterilizable ultrasound probe is described in the US patent N° 5 678 551. This ultrasound probe includes an electrical cable having first end and second end, an ultrasound transducer connected to the first end of the cable and a connector assembly connected to the second end of the cable. The connector assembly includes a connector housing having a cable opening and a mating connector opening, a connector body assembly mounted within the connector housing, a removable cover assembly sealed to the connector housing and covering the mating connector opening, and a strain relief assembly for sealing the cable opening between the cable and the connector housing. When the removable cover assembly is sealed to the connector housing, the connector assembly is immersible in a liquid, such as a sterilization fluid. The cover assembly is removed from the connector housing for operation of the ultrasound probe with an imaging system.
An ultrasonic probe connector structure is also described in the application EP 98 309 612.4. This connector structure comprises a printed circuit board including cable lands for connecting coaxial cables of the ultrasonic probe. The connector lands have patterns for the connector pin places of the ultrasonic observation device and a wiring pattern for electrically connecting said connector lands with said cable lands. The system has a pressure abutment. Connector pins are fixed to the connector housing so that they can coincide with the connector pin places of said ultrasonic observation device repectively. Pressure retainer means may fixedly press said connector pins to said connector lands of said printed circuit board by means of said pressure abutment connector.

In accordance with the principles of the present invention, an ultrasonic probe connector enclosure is provided that seals-the connector in a simple and reliable manner such that the connector may be completely submersed in a sterilization liquid without causing damage to the components of the connector. In a preferred embodiment the present invention is comprised of an enclosure for enclosing the connector and a portion of the connector cable in an enclosure that has a liquid tight seal.

In the drawings:
FIGS. 1a-1b illustrate a top and front view of one embodiment of the present invention;
FIG. 2 illustrates in cross section a sealing technique utilized by the present invention;
FIGS. 3a-3b illustrate top and front views of a sealing device of the present invention; and

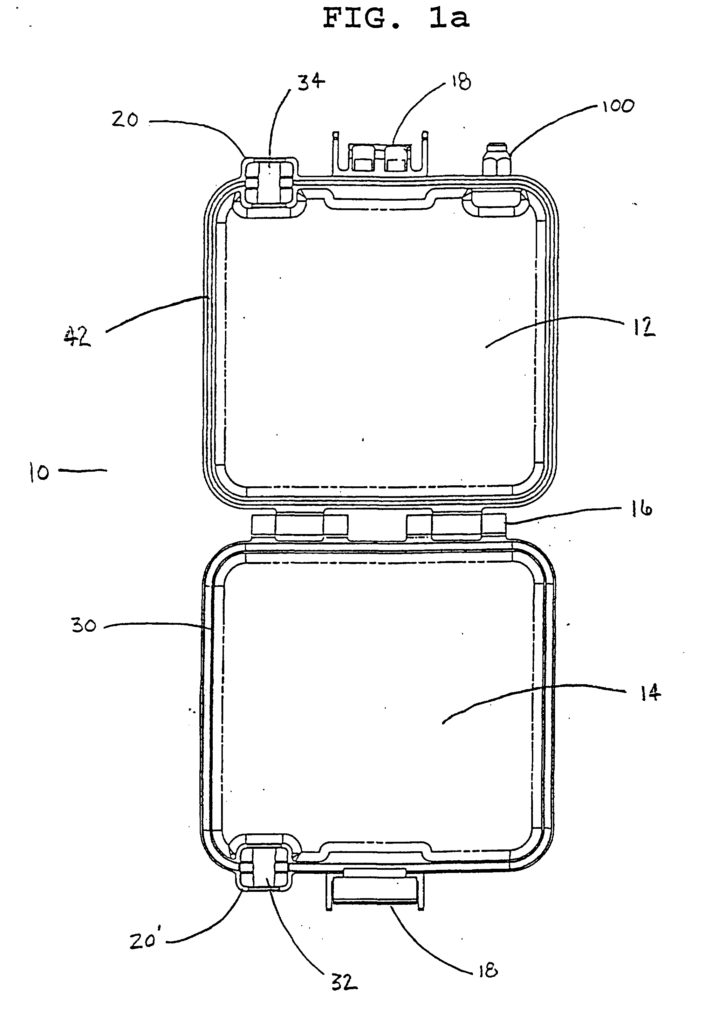

Referring first to FIGS. 1a-1b, a connector enclosure is shown that solves the problems encountered when a connector is submerged in the liquid sterilization environment by enclosing the connector and a portion of the connector cable in an enclosure that has a liquid tight seal.

As shown in Figures 1a-1b, the connector enclosure IO of the present invention is comprised of a top enclosure half 12 and a bottom enclosure half 14. Top enclosure half ₁2 and bottom enclosure half 14 may be latched together with a common latching device 18. Enclosure halves 12 and 14 are shown connected by hinging device 16; however, a latching device may used in its place.

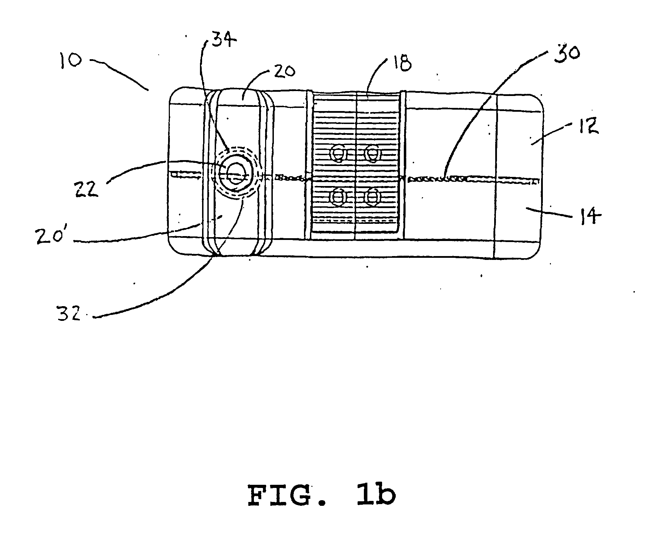

Figure 1 a shows a top view of connector enclosure IO with notch 20 on top enclosure half 12. Figure 1 b shows notch 20' on bottom enclosure half 14, which is matched to mate with notch 20 when the enclosure halves are latched. Figure 1 b shows opening 22 located between notches 20 and 20' for holding and sealing the area around the probe cable. A resilient gasket 30 is also shown in Figures 1a-1b for providing a liquid tight seal between enclosure halves 12 and 14.

In a preferred embodiment, a check valve 100 is located on either top or bottom enclosure halve. The checkvalve provides the user with the ability to test gasket 30 for leaks. To test the seal, a hand pump and pressure gage are attached to an inbound port of the check valve to pressurize the enclosure to a desired pressure. The user may then observe stability of the pressure over a given time period to determine if a leak exists.

When a probe requires sterilization, the user must first orient the probe connector (not shown) into top half 12 or bottom half 14 such that the probe cable (not shown) aligns with and lays in one-half of opening 22. Once the connector and cable are properly positioned, the enclosure half without the connector may be rotated and closed over the connector by hinging device 16, and latched with latch 18. When latch 18 is latched, gasket 30, top enclosure half 12, bottom enclosure half 14, and opening 22 create a liquid tight seal around the connector and cable.

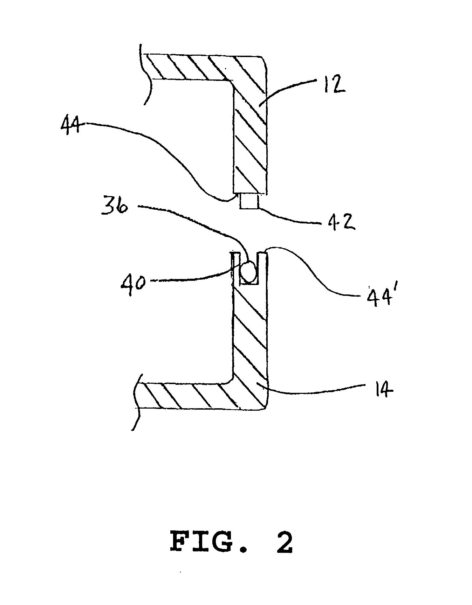

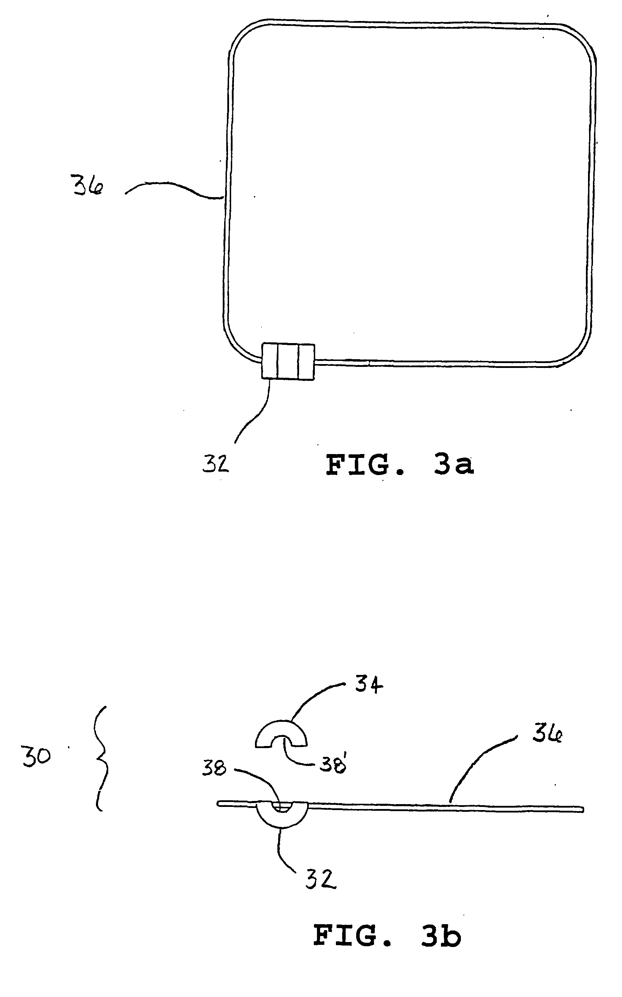

Turning now to FIG. 2, groove 40 is shown in a cross section view of bottom enclosure half 14 and tongue 42 is shown in a cross section view of top enclosure half 12. Tongue 42 is located on mating surface 44 and groove 40 is located on opposing mating surface 44'. During assembly of enclosure connector 10, the body 36 of gasket 30 (See FIG. 3a) is positioned in groove 40. The outside diameter of body 36 is slightly larger than the diameter of groove 40 such that body 36 is snugly held in place when positioned in groove 40 (not shown). As top enclosure half 12 and bottom enclosure half 14 are latched together, body 36 is compressed between groove 40 and tongue 42 and a liquid tight seal is formed between the enclosure halves (not shown).

Turning now to FIGS. 3a and 3b, top and front views of gasket 30 are shown. Gasket 30 is comprised of bottom sealing portion 32, top sealing portion 34, and body 36. Figure 3a shows body 36 shaped to conform to the circumference of bottom enclosure half 14. FIG. 3a shows bottom sealing portion 32 molded into body portion 36 such that a one piece gasket is formed. Bottom sealing portion 32 is shaped to fit snugly in notch 20' of bottom enclosure 14.

Bottom sealing portion 32 is semi-circular in shape having a solid volumetric body with an inside diameter 38 shaped to fit snugly against the cable of a probe connector (not shown). Top sealing portion 34, as shown in FIG. 3b, is matched in shape and size to bottom sealing portion 32 and has identical inside diameter 38. However, top sealing portion 34 is not integrated into body 36. Rather, top portion 34 is positioned in notch 20 of top enclosure half 14 (See FIG. 1 b). When a connector and cable are positioned in enclosure 10 and the cable is positioned in opening 22, a liquid tight seal is formed when top enclosure half 12 is latched to bottom enclosure half 14 by latch 18. Although body 36 is described as being located in bottom enclosure half 14, top enclosure half 12 may be readily configured to accept body 36.

Thus, the present invention provides a connector enclosure used for protecting sensitive electronic components of the connector during the sterilization process of ultrasonic probes. Moreover, the present invention may be used on any type of ultrasonic probe and is not limited to intra-operative probes.

## Claims

1. An enclosure, for enclosing and sealing a connector and a portion of a cable of an ultrasonic probe assembly in a liquid tight manner when said probe is submerged in a sterilization liquid, comprising:
a housing, for enclosing said connector and said portion of said cable, wherein said housing is comprised of a top enclosure half (12) and a bottom enclosure half (14) for receiving said connector, each enclosure half (12,14) having opposing mating surfaces (44,44'), while said portion of cable is positioned in an opening located between matched mating notches (20,20'), formed in said top enclosure half (12) and said bottom enclosure half (14), so that the connector and cable are separable and removable from said housing when said top enclosure half (12) is open;
sealing means for sealing said mating surfaces; and
a latch (18) for latching said top enclosure halt (12) to said bottom enclosure half (14).

2. The enclosure of Claim 1, wherein said sealing means is comprised of a gasket (30).

3. The enclosure of Claim 2, wherein said gasket (30) is made from a resilient material.

4. The enclosure of one of Claims 2 or 3, wherein said gasket (30) is sized for compression between a groove (40) circumferentially located on said mating surface (44,44') of said bottom enclosure half (12) and a tongue (42) circumferentially located on said mating surface (44') of said top enclosure half (14).

5. The enclosure of any one of Claims 2 to 4, wherein said gasket (30) includes a bottom sealing portion (32) and a top sealing (34) portion for sealing said cable in a liquid tight manner while said cable is positioned in the opening (22) located between said top enclosure half (12) and said bottom enclosure half (14).

6. The enclosure of Claim 5, wherein said bottom sealing (32) portion is affixed to said gasket (30) and located in a notch (20') in said bottom enclosure half (14).

7. The enclosure of Claim 6, wherein said top sealing portion (34) is located in a notch (20) in said top enclosure half.

8. The enclosure of any one of Claims 2 to 7, wherein said gasket (30) includes a cable sealing portion for creating a liquid tight seal between said cable and said enclosure halves.

9. The enclosure of one of Claim 2 to 8, wherein said cable sealing portion is comprised of a first and second portion.

10. The enclosure of Claim 9, wherein said first cable sealing portion is affixed to said gasket and located in a notch on one of said enclosure halves and said second cable sealing portion is located in a notch in said opposing enclosure half.

## Patentansprüche

1. Gehäuse zum Umschließen und flüssigkeitsundurchlässigen Abdichten eines Steckverbinders und eines Teils eines Kabels einer Ultraschallsondenbaugruppe, wenn die genannte Sonde in eine Sterilisationsflüssigkeit eingetaucht wird, wobei das Gehäuse Folgendes umfasst:
ein Gehäuse zum Umschließen des genannten Steckverbinders und des genannten Teils des genannten Kabels, wobei das genannte Gehäuse aus einer oberen Gehäusehälfte (12) und einer unteren Gehäusehälfte (14) zur Aufnahme des genannten Steckverbinders besteht und jede Gehäusehälfte (12, 14) einander gegenüberliegende Passflächen (44, 44') hat, während der genannte Teil des Kabels in einer Öffnung positioniert wird, die sich zwischen den übereinstimmenden Kerben (20, 20') befindet, welche in die genannte obere Gehäusehälfte (12) und die genannte untere Gehäusehälfte (14) eingeformt sind, so dass der Steckverbinder und das Kabel trennbar und abnehmbar von dem genannten Gehäuse sind, wenn die genannte obere Gehäusehälfte (12) geöffnet ist;
Abdichtungsmittel zum Abdichten der genannten Passflächen; und
eine Verriegelung (18) zum Verriegeln der genannten oberen Gehäusehälfte (12) mit der genannten unteren Gehäusehälfte (14).

2. Gehäuse nach Anspruch 1, wobei die genannten Abdichtungsmittel eine Dichtung (30) umfassen.

3. Gehäuse nach Anspruch 2, wobei die genannte Dichtung (30) aus einem elastischen Material besteht.

4. Gehäuse nach einem der Ansprüche 2 oder 3, wobei die genannte Dichtung (30) so bemessen ist, dass sie zwischen einer Nut (40), die um den Umfang der genannten Passfläche (44') der genannten unteren Gehäusehälfte (14) herum angeordnet ist, und einer Zunge (42), die um den Umfang der genannten Passfläche (44) der genannten oberen Gehäusehälfte (12) herum angeordnet ist, zusammengedrückt wird.

5. Gehäuse nach einem der Ansprüche 2 bis 4, wobei die genannte Dichtung (30) einen unteren Abdichtungsteil (32) und einen oberen Abdichtungsteil (34) zum flüssigkeitsundurchlässigen Abdichten des genannten Kabels umfasst, während das genannte Kabel in der Öffnung (22) liegt, die sich zwischen der genannten oberen Gehäusehälfte (12) und der genannten unteren Gehäusehälfte (14) befindet.

6. Gehäuse nach Anspruch 5, wobei der genannte untere Abdichtungsteil (32) an der genannten Dichtung (30) befestigt ist und sich in einer Kerbe (20') in der genannten unteren Gehäusehälfte (14) befindet.

7. Gehäuse nach Anspruch 6, wobei sich der obere Abdichtungsteil (34) in einer Kerbe (20) in der genannten oberen Gehäusehälfte (12) befindet.

8. Gehäuse nach einem der Ansprüche 2 bis 7, wobei die genannte Dichtung (30) einen Kabelabdichtungsteil zum Schaffen einer flüssigkeitsundurchlässigen Abdichtung zwischen dem genannten Kabel und den genannten Gehäusehälften umfasst.

9. Gehäuse nach einem der Ansprüche 2 bis 8, wobei der genannte Kabelabdichtungsteil aus einem ersten und einem zweiten Teil besteht.

10. Gehäuse nach Anspruch 9, wobei der erste Kabelabdichtungsteil an der genannten Dichtung befestigt ist und sich in einer Kerbe in einer der genannten Gehäusehälften befindet und der genannte zweite Kabelabdichtungsteil sich in einer Kerbe in der genannten gegenüberliegenden Gehäusehälfte befindet.

## Revendications

1. Enveloppe, pour envelopper et sceller de manière étanche un connecteur et une partie d'un câble d'un montage de sonde ultrasonore lorsque ladite sonde est immergée dans un liquide de stérilisation, comprenant :
un boîtier, pour envelopper ledit connecteur et ladite partie dudit câble, dans laquelle ledit boîtier est composé d'une moitié d'enveloppe supérieure (12) et d'une moitié d'enveloppe inférieure (14) pour recevoir ledit connecteur, chaque moitié d'enveloppe (12, 14) comportant des surfaces d'ajustement opposées (44, 44'), tandis que ladite partie de câble est positionnée dans une ouverture située entre des encoches d'ajustement adaptées (20, 20'), formées dans ladite moitié d'enveloppe supérieure (12) et ladite moitié d'enveloppe inférieure (14), de sorte que le connecteur et le câble sont séparables et amovibles dudit boîtier lorsque ladite moitié d'enveloppe supérieure (12) est ouverte ;
un moyen de scellement pour sceller lesdites surfaces d'ajustement, et
un verrou (18) pour verrouiller ladite moitié d'enveloppe supérieure (12) à ladite moitié d'enveloppe inférieure (14).

2. Enveloppe suivant la revendication 1, dans laquelle ledit moyen de scellement est composé d'un joint d'étanchéité (30).

3. Enveloppe suivant la revendication 2, dans laquelle ledit joint d'étanchéité (30) est fait en un matériau élastique.

4. Enveloppe suivant l'une quelconque des revendications 2 ou 3, dans laquelle ledit joint d'étanchéité (30) est calibré pour compression entre une rainure (40) située de manière circonférentielle sur ladite surface d'ajustement (44, 44') de ladite moitié d'enveloppe inférieure (12) et une languette (42) située de manière circonférentielle sur ladite surface d'ajustement (44') de ladite moitié d'enveloppe supérieure (14).

5. Enveloppe suivant l'une quelconque des revendications 2 à 4, dans laquelle ledit joint d'étanchéité (30) comprend une partie de scellement inférieure (32) et une partie de scellement supérieure (34) pour sceller de manière étanche ledit câble tandis que ledit câble est positionné dans l'ouverture (22) située entre ladite moitié d'enveloppe supérieure (12) et ladite moitié d'enveloppe inférieure (14).

6. Enveloppe suivant la revendication 5, dans laquelle ladite partie de scellement inférieure (32) est attachée audit joint d'étanchéité (30) et située dans une encoche (20') dans ladite moitié d'enveloppe inférieure (14).

7. Enveloppe suivant la revendication 6, dans laquelle ladite partie de scellement supérieure (34) est située dans une encoche (20) dans ladite moitié d'enveloppe supérieure.

8. Enveloppe suivant l'une quelconque des revendications 2 à 7, dans laquelle ledit joint d'étanchéité (30) comprend une partie de scellement de câble pour créer un scellement étanche entre ledit câble et lesdites moitiés d'enveloppe.

9. Enveloppe suivant l'une quelconque des revendications 2 à 8, dans laquelle ladite partie de scellement de câble est composée d'une première et d'une deuxième parties.

10. Enveloppe suivant la revendication 9, dans laquelle ladite première partie de scellement de câble est attachée audit joint d'étanchéité et située dans une encoche sur une desdites moitiés d'enveloppe et ladite deuxième partie de scellement de câble est située dans une encoche dans ladite moitié d'enveloppe opposée.
